(19) Europäisches Patentamt
European Patent Office
Office européen des brevets

(11) Publication number: 0 087 388 B1

(12) # EUROPEAN PATENT SPECIFICATION

(45) Date of publication of the patent specification: **25.06.86**

(51) Int. Cl.[4]: **G 03 C 7/38,** C 07 D 421/04, C 07 D 417/04, C 07 D 413/04

(21) Application number: **83810046.9**

(22) Date of filing: **04.02.83**

(54) **Pyrazolone couplers and colour photographic silver halide recording material containing them.**

(30) Priority: **10.02.82 GB 8203932**

(43) Date of publication of application: **31.08.83 Bulletin 83/35**

(45) Publication of the grant of the patent: **25.06.86 Bulletin 86/26**

(84) Designated Contracting States: **BE CH DE FR GB IT LI**

(56) References cited:
**DE - A - 2 542 651**
**FR - A - 2 322 390**

(73) Proprietor: **CIBA-GEIGY AG, Klybeckstrasse 141, CH-4002 Basel (CH)**

(72) Inventor: **Webb, Terence Charles, 5 The Avenue, Danbury Essex (GB)**
Inventor: **Long, William Edward, 129 Shevon Way, Brentwood Essex (GB)**

EP 0 087 388 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to pyrazolone colour couplers.

Pyrazolone compounds have long been used as four-equivalent colour couplers in colour photographic materials. However, it has been found that these compounds are very susceptible to attack by formaldehyde and other aldehydes. Synthetic plastics materials have almost entirely replaced leather as camera cases and camera accessory bags. Unfortunately a number of these plastics materials are made by using formaldehyde or other aldehydes for example formaldehyde-melamine resins and phenol-formaldehyde resins. Such plastics materials tend to produce a small but significant amount of formaldehyde vapour continuously. When colour photographic material which comprises in a layer of the material a four-equivalent pyrazolone colour coupler is stored in a bag or case made from a plastics material which comprises formaldehyde or another aldehyde it has been found that the formaldehyde or other aldehydes have a deleterious effect on the pyrazolone colour coupler which is shown by a degradation in the dye produced by the coupling reaction when such photographic material has been exposed and colour processed. However, it has been found that 2-equivalent pyrazolone colour couplers are not affected in the same way by formaldehyde or other aldehydic vapours. But it has proved difficult to prepare on a commercial scale 2-equivalent pyrazolone colours couplers which exhibit sufficiently good coupling activity to be used in colour photographic material.

2-equivalent pyrazolone colour couplers, wherein the pyrazolone leaving group is connected with a pivaloyl- or benzoylacetanitide moiety, are known for example from DE-A-2 542 651 and FR-A-2 322 390.

We have found a novel class of 2-equivalent pyrazolone colour couplers which may be prepared on a commercial scale and which have a good coupling activity. Moreover, the dyes produced on the colour coupling of the pyrazolone have good light stability.

According to the present invention there is provided a pyrazolone colour coupler of the formula

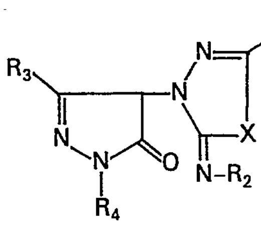

(1)

wherein X is -S-, -O- or -Se-, $R_1$ is hydrogen, chlorine, bromine or iodine or optionally substituted alkyl, phenyl, alkoxy, phenoxy, acyloxy, alkylthio or phenylthio, $R_2$ is -CO-alkyl, $-CO-C_6H_5$, $-CONH-C_6H_5$, -CONH-alkyl, $-SO_2$-alkyl or $-SO_2-C_6H_5$, wherein the alkyl and $C_6H_5$-radicals are optionally substituted, $R_3$ is alkyl, phenyl, -NH-alkyl, $-NH-C_6H_5$, -NHCONH-alkyl, $-NHCONH-C_6H_5$ or -NHCO-alkyl, wherein the alkyl and $C_6H_5$-radicals are optionally substituted, $R_4$ is hydrogen or substituted alkyl or phenyl, at least in one of the alkyl or phenyl groups of $R_3$ or $R_4$ being present a ballasting group B.

X in the compounds of the formula (1) denotes -S-, -O- or -Se-. Preferebly X is -S-.

Substituent $R_1$ is hydrogen or halogen such as chlorine, bromine or iodine. $R_1$ further denotes alkyl, preferably having 1 to 6 carbon atoms such as methyl, ethyl, propyl, butyl, pentyl and hexyl as well as isomers thereof. The alkoxy and alkylthio radicals $R_1$ are preferably those which can be derived from the alkyl radicals $R_1$ just mentioned. Phenyl, phenoxy and phenylthio are further meanings of $R_1$. $R_1$ also denotes acyloxy, preferably carboxylic acid acyl of the formula

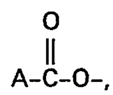

where A is aryl, preferably phenyl, or alkyl having preferably 1 to 6 carbon atoms.

Substituent $R_1$ is optionally further substituted by other phenyl groups or also by alkyl, alkoxy,

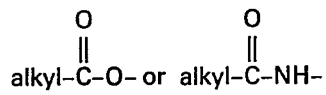

group, wherein the alkyl moieties preferably contain 1 to 4 carbon atoms. Further suitable substituents on $R_1$ are halogen atoms such as chlorine and bromine.

Substituent $R_2$ represents an acyl or acylamino group preferably having the formulae -CO-alkyl, $-SO_2$-alkyl, -CONH-alkyl, wherein alkyl contains preferably 1 to 6 carbon atoms (suitable alkyl groups are mentioned above in the definitions of $R_1$), or phenyl, $-CO-C_6H_5$, $-SO_2-C_6H_5$ or $-CONH-C_6H_5$.

The alkyl and phenyl moieties present in $R_2$ are optionally substituted by further alkyl or hydroxyalkyl radicals,

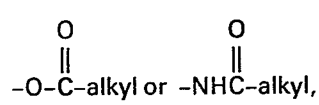

the alkyl moieties in these groups preferably containing 1 to 4 carbon atoms, or halogen such as chlorine or bromine.

Substituent $R_3$ denotes alkyl, -NH-alkyl, -NHCONH-alkyl or -NHCO-alkyl, the alkyl radicals having preferably 1 to 6 carbon atoms (suitable alkyl radicals are mentioned above), further phenyl, $-NH-C_6H_5$, $-NHCONH-C_6H_5$ or $-NHCO-C_6H_5$.

The alkyl and phenyl moieties present in $R_3$ are optionally further substituted by electronegative groups such as halogen, preferably chlorine or bromine, or cyano, or hydroxyalkyl,

$$-O-\overset{\overset{\displaystyle O}{\|}}{C}-\text{alkyl},$$

-CONH-alkyl or -NHCO-alkyl, the alkyl radicals comprising preferably 1 to 6 carbon atoms.

Substituent $R_4$ is hydrogen or substituted alkyl or, preferably, substituted phenyl. Suitable substituents on phenyl and the alkyl radicals are halogen such as chlorine or bromine, alkyl, hydroxylalkyl or alkyl,

$$-O-\overset{\overset{\displaystyle O}{\|}}{C}-$$

further $-SO_2NHR_5$, $-NHCOR_5$ or $-NHSO_2R_5$, where $R_5$ is alkyl or phenyl which is optionally further substituted by alkoxy such as methoxy or ethoxy, or halogen. All the alkyl moieties mentioned contain as a rule 1 to 6, preferably 1 to 4 carbon atoms. At least one of the alkyl or phenyl groups of $R_3$ or $R_4$ contain a ballasting group B. Preferably B comprises long chain alkyl groups having more than 10 carbon atoms, preferably up to 30 carbon atoms, such as decyl, dodecyl, hexadecyl, heptadecyl, octadecyl, eicosyl, tetracosyl or triacontyl, or more preferably B represents an aryloxy group substituted by tertiary alkyl radicals having 4 to 8 carbon atoms, for example, ditertiary pentyl substituted phenoxy.

In a preferred class of the couplers of the formula (1), $R_1$ is hydrogen, chlorine, bromine or iodine or alkyl, alkoxy or alkylthio, wherein the alkyl moiety contains 1 to 6 carbon atoms, or phenyl, phenoxy, phenylthio or carboxylic acid acyl, $R_2$ is -CO-alkyl, -CONH-alkyl or $-SO_2$-alkyl, wherein the alkyl moiety contains 1 to 6 carbon atoms, or $-C_6H_5$, $-CONH-C_6H_5$ or $-SO_2-C_6H_5$, and wherein the alkyl and phenyl moieties are unsubstituted or substituted by alkyl or hydroxyalkyl, carbalkoxy, -NHCO-alkyl or halogen, the alkyl radicals containing 1 to 4 carbon atoms, $R_3$ is alkyl, -NH-alkyl or -NHCONH-alkyl, the alkyl moieties containing 1 to 6 carbon atoms, or phenyl, $-NH-C_6H_5$, $-NHCONH-C_6H_5$ or $-NHCO-C_6H_5$, wherein phenyl and the alkyl moieties are unsubstituted or substituted by halogen, cyano, hydroxyalkyl, carbalkoxy, -CONH-alkyl or -NHCO-alkyl, the alkyl radicals containing 1 to 6 carbon atoms, $R_4$ is hydrogen, alkyl having 1 to 6 carbon atoms or phenyl, where the alkyl moieties and phenyl are substituted by halogen, alkyl, hydroxyalkyl or carbalkoxy, the alkyl moiety containing 1 to 4 carbon atoms, or $-SO_2NHR_5$, $-NHCOR_5$ or $-NHSO_2R_5$, wherein $R_5$ is alkyl having 1 to 6 carbon atoms or phenyl, which radicals are unsubstituted or substituted by methoxy, ethoxy or halogen, at least in one of the alkyl or phenyl groups of $R_3$ or $R_4$ being present a ballasting group B, and X is as defined above.

More preferably in the couplers of the formula (1), X is -O- or -S-, $R_1$ is alkyl or alkoxy, wherein the alkyl moiety contains 1 to 6 carbon atoms, or phenyl or phenoxy, $R_2$ is -CO-alkyl or $-SO_2$-phenyl, wherein phenyl and the alkyl moieties are unsubstituted or substituted by alkyl or hydroxyalkyl each having 1 to 4 carbon atoms, or halogen, $R_3$ is alkyl, phenyl, $-NH-C_6H_5$, $-NHCONH-6_6H_5$ or $-NHCO-C_6H_5$, wherein phenyl and the alkyl moieties are unsubstituted or substituted by halogen, cyano, hydroxyalkyl, carbalkoxy, -CONH-alkyl or -NHCO-alkyl, the alkyl radicals containing 1 to 6 carbon atoms, $R_4$ is phenyl substituted by halogen, $-SO_2NHR_5$, $-NHCOR_5$ or $-NHSO_2R_5$, wherein $R_5$ is alkyl having 1 to 6 carbon atoms, at least in one of the alkyl or phenyl groups of $R_3$ or $R_4$ being present a ballasting group B.

It is to be understood that the compounds of the formula (1) may also be present as tautomers having the formula

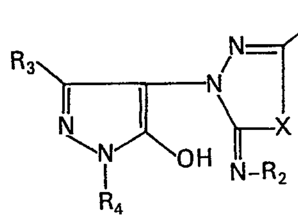

(2)

wherein $R_1$, $R_2$, $R_3$, $R_4$ and X have the meanings assigned to them above.

These compounds may be synthesised by reaction of a chloro or bromo pyrazolone of the formula

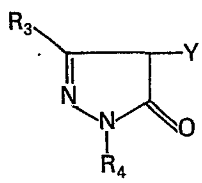

(3)

wherein $R_3$ and $R_4$ have the meanings assigned to them above, and Y is chlorine or bromine, with an oxa, thia- or selenadiazole compound of the formula

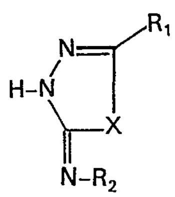

(4)

wherein X, $R_1$ and $R_2$ have the meanings assigned to them above, in the presence of a basic catalyst such as sodium or potassium hydroxide, carbonate or alkoxide, or an amine base, such as an excess of the compound of formula (4), either in the melt or in solution or suspension in a solvent, for example acetonitrile.

Alternatively, and this is preferably, compounds of the formula (1) may be prepared from the reaction of an acyclic precursor already containing the oxa, thia- or selenadiazole ring of the formula

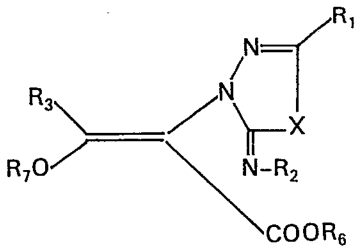

(5)

wherein $R_6$ and $R_7$ are each hydrogen, alkyl or phenyl, and $R_1$, $R_2$, $R_3$ and X are groups as defined above, or groups which may be transformed into such a group, with a substituted hydrazine of the formula

$H_2N\text{-}NHR_4$ (6)

wherein $R_4$ has the meaning assigned to it above, or is a group which may be transformed into such a group either in a melt, or in a suitable solvent, either in the presence of an acidic catalyst such as acetic acid, or of a basic catalyst such as pyridine or sodium ethoxide, or without catalyst at all, at room temperature or a higher temperature.

Preferably the above reaction is carried out in the presence of an acid catalyst and most preferably this is in acetic acid.

Preferably the above reaction is carried out in a solvent and most preferably this solvent is an (alkyl or substituted alkyl) alcohol. Suitable alcohols are ethanol, propanol and ethoxy-ethanol.

Preferably the above reaction is carried out in an alcoholic solvent and the reaction temperature is at the boiling point of the solvent.

An example of a group which can be transformed to a group as defined in the compounds of the formula (1) is the transformation of $-NO_2$ to $-NH_2$ followed by conversion of $-NH_2$ to $-NHCOB$, where B is as defined above, by reaction with an acid chloride.

Another example of a transformable group is the protection of an hydroxy group in form of an ester group. The ester group can then be removed after the main reaction has taken place.

Thus, for preparing a pyrazolone where $R_3$ is $-NH\text{-}C_6H_5$, a suitable compound of the formula (5) is the compound of the formula

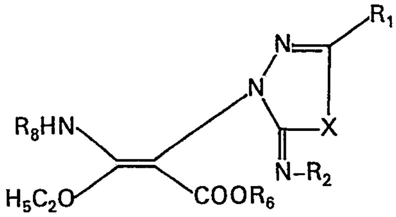

(7)

wherein $R_1$, $R_2$, $R_6$ and X have the meanings assigned to them above, and $R_8$ is phenyl, which may be substituted for example by chlorine, bromine, cyano, alkoxy, alkyl, amido or an ester group, or by a ballast group as defined above.

It is to be understood that compounds of the formula (7) may exist in the tautomeric form of the formula

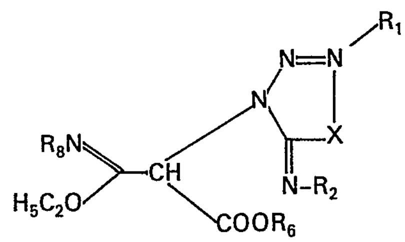

(8)

wherein $R_1$, $R_2$, $R_6$, $R_8$ and X have the meanings assigned to them above.

Compounds of the formula (7) or (8) may be prepared by reaction of an oxa-, thia- or selenadiazole of the formula (4) with a bromo or chloro compound of the formula

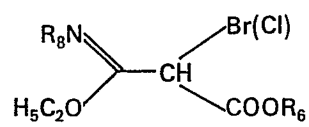

(9)

wherein $R_6$ and $R_8$ have the meaning assigned to them above, in the presence of a basic catalyst such as sodium or potassium hydroxide, carbonate or alkoxide, or an amine base such as an excess of the compound of the formula (4).

Compounds of the formula (9) may be prepared as described in GB-A-1,325,828; GB-A-1,129,133; GB-A-1,129,334 or GB-A-1,455,967. Compounds of the formula (4) may be prepared as described in GB-A-1,566,618.

Similarly, when the group $R_2$ is alkyl, a suitable precursor of the formula (5) is the compound of the formula

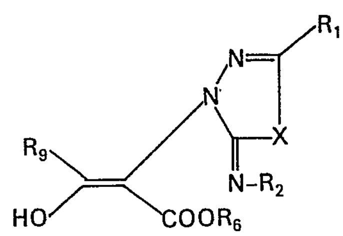

(10)

wherein $R_1$, $R_2$, $R_6$ and X have the meanings assigned to them above, and $R_9$ is alkyl or cycloalkyl, which radicals may be substituted.

Again, it is to be understood that the compounds of the formula (10) may exist in the tautomeric form of the formula

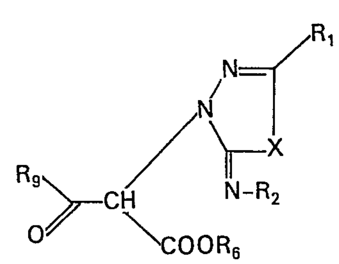

(11)

wherein $R_1$, $R_2$, $R_6$, $R_9$ and X have the meanings assigned to them above.

Compounds of the formula (10) or (11) may be prepared by reaction of a chloro or bromo ester of the formula

$R_9COCHCOOR_6$ (12)
|
Y

wherein $R_6$ and $R_9$ have the meanings assigned to them above, and Y is chlorine or bromine with an oxa- thia- or selenadiazole of the formula (4), in the presence of a base such as sodium or potassium hydroxide, carbonate or alkoxide, in a suitable solvent such as acetonitrile.

The chloro- or bromo-compounds of the formula (12) may be prepared from the corresponding ketoesters by chlorination or bromination by methods well known in the literature.

Particularly useful pyrazolone compounds of the formula (1) are the compounds of the formulae

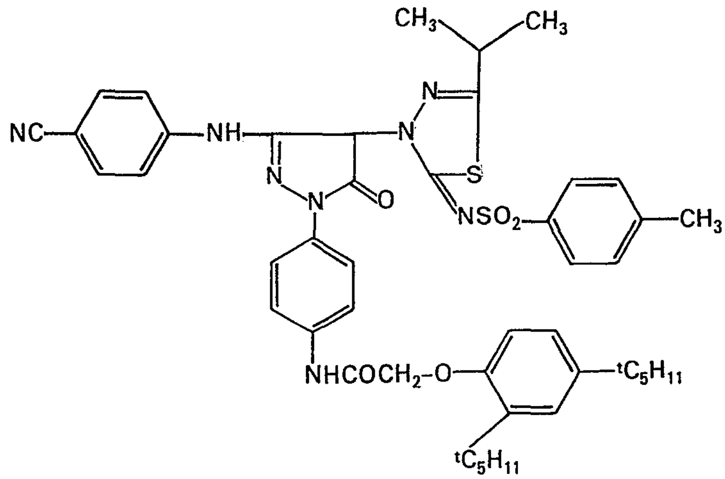

(13)

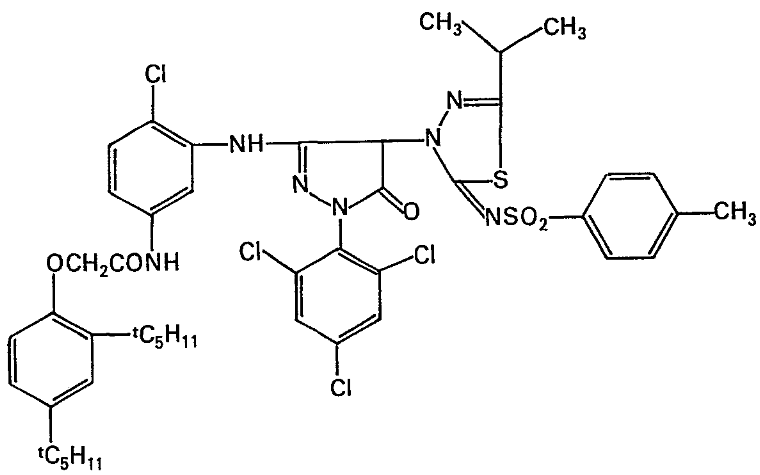

(14)

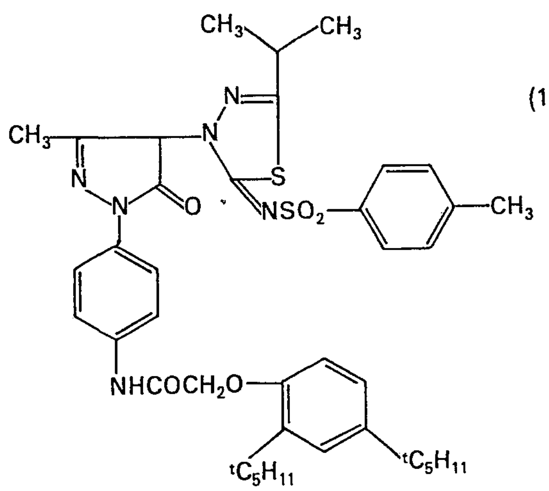

(15)

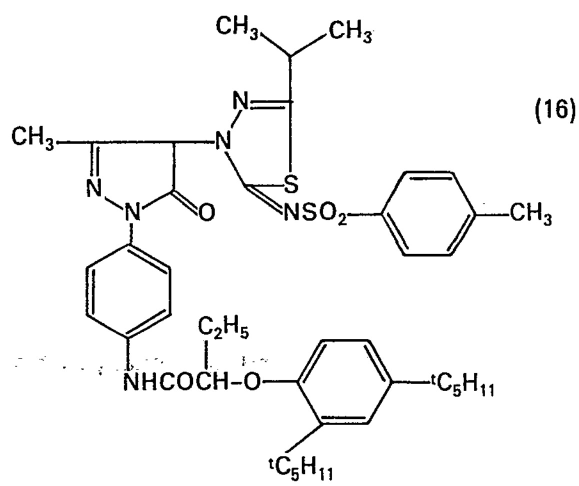

(16)

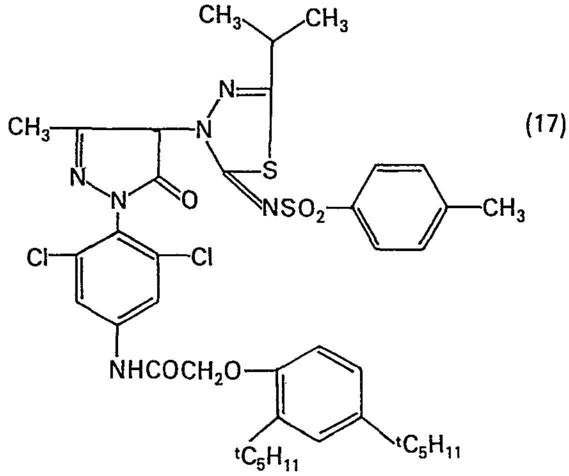

(17)

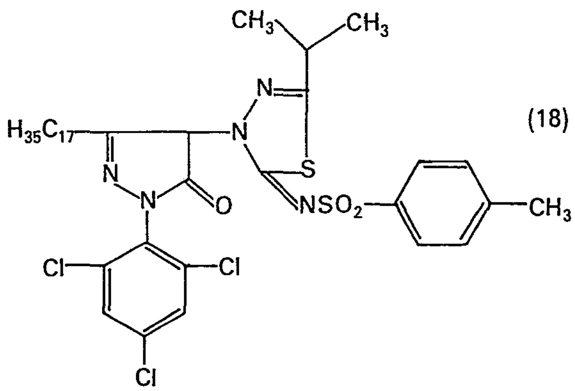

(18)

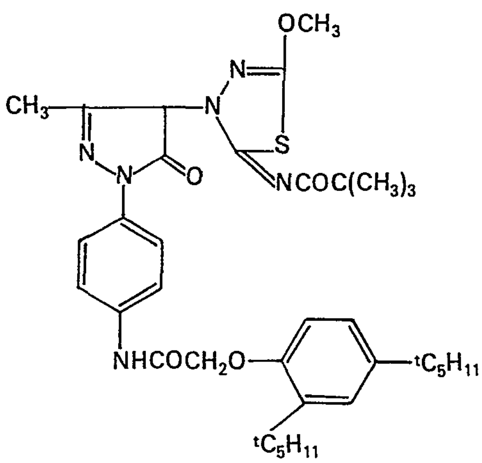

(19)

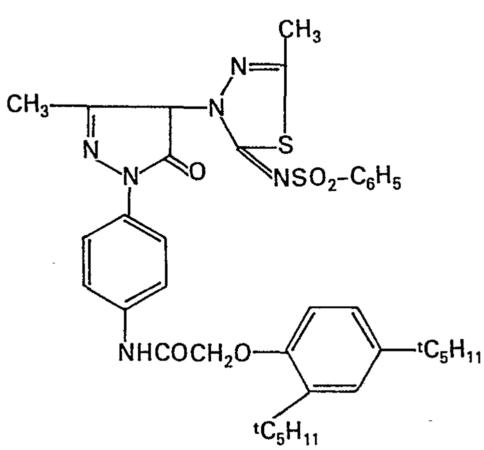

(20)

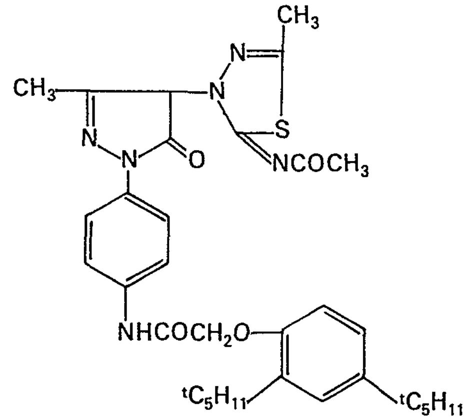

(21)

and

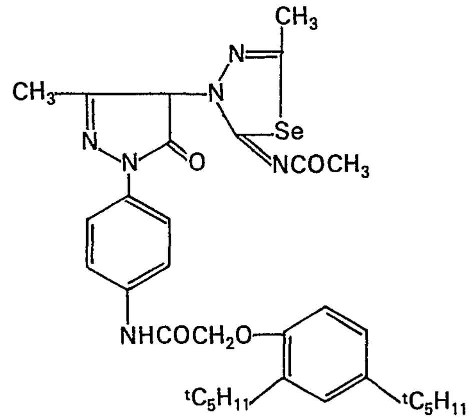

(22)

According to another aspect of the present invention, there is provided colour photographic silver halide material which comprises in at least one silver halide layer a pyrazolone colour coupler of the formula (1).

The compounds of the formula (1) wherein $R_3$ is a -NH- substituent group wherein the substituent group comprises a ballasting group B and $R_4$ is a substituent phenyl group yield on coupling pure magenta dyes.

The compounds of the formula (1) where the ballasting group B is present in $R_4$ yield on coupling dyes which are reddish. These compounds find use as colour couplers in chromogenic monochrome film material wherein the dyes form a monochrome image from which a black and white print is obtained.

Photographic material which comprises in a layer thereof a compound of the formula (1) is processed after exposure by a colour development process using a primary aromatic amine colour developing agent of known type. As usual in colour development processes the primary aromatic amine develops the latent silver image to form a silver image and becomes oxidised, and the oxidised colour developer couples which the pyrazolone colour coupler of the formula (1) to form a magenta or reddish dye image.

After the colour development reaction to form the dye image the unexposed silver halide in the photographic material is removed using a silver halide solvent and the silver image is removed using a silver bleach bath. The silver halide and the

silver may be removed by use of a simple bleach-fix (blix) bath.

Suitable primary aromatic amine colour developing agents are p-phenyl-enediamine compounds, for example 4-amino-N,N-dimethylaniline hydrochloride, 4-amino-N,N-diethylaniline hydrochloride, 4-amino-3-methyl-N,N-diethylaniline hydrochloride and p-aminophenol compounds for example a p-aminophenol itself and 2,6-dichloro-4-aminophenol.

The following Example will serve to illustrate the invention.

Example 1:

Synthesis of the compound of the formula (15): 2-p-Toluenesulfonylimino-5-isopropyl-1,3,4-thiadiazole (14.55 g) and solid potassium hydroxide (3.23 g) in toluene (150 ml) are heated under a Dean-Stark trap for 7 hours and then cooled. Acetonitrile (100 ml) and ethyl-2-chloro acetoacetate (10 ml) are added, and the reaction heated to reflux for 5 hours during which time a solid appears. Water (250 ml) is added, the organic layer separated and washed with water, and the aqueous layers extracted with ether. The combined organic layers are dried and evaporated, and the resulting oil is added to petrol (bp 80–100°C) (200 ml) with ethyl acetate (10 ml) and heated to boiling with charcoal. After filtration, 12.8 g of the product crystallise out on cooling (melting point 102–105°C). NMR analysis shows it to have the enol structure of the formula

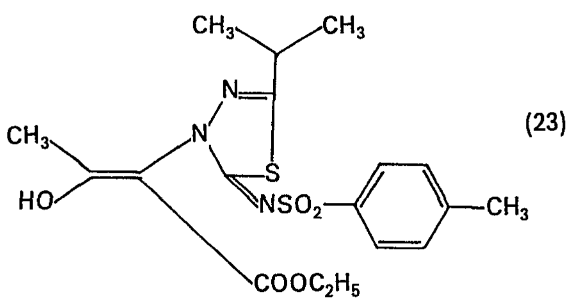

(23)

The above compound (2.13 g) and 4-nitro phenyl hydrazine (0.77 g) in acetic acid (10 ml) and ethanol (5 ml) is heated to reflux for 3 hours and cooled. 2.68 g of the solid are filtered off (melting point 211–216°C).

NMR analysis shows it to have the following structure

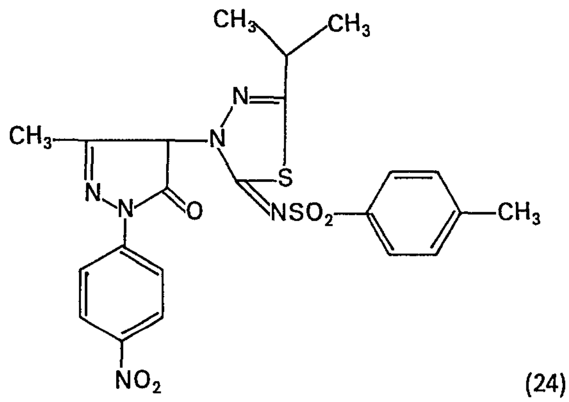

(24)

A sample of this compound (2.63 g) is hydrogenated at room temperature in ethanol (100 ml) over a hydrogenation catalyst (10% palladium on charcoal) for 3 days. The catalyst is filtered off, and the solvent evaporated to give an oil, which is dissolved in tetrahydrofuran and pyridine, and reacted with the acid chloride from di-t-amylphenoxyacetic acid (1.49 g) for 3 hours. The product is added to hydrochlorid acid, and extracted with ether. This ether phase is dried and evaporated, and 1.04 g of the compound of the formula (15) crystallise on triturating with methanol (melting point 168–174°C).

Example 2:

Synthesis of the compound of the formula (16):

A sample of the nitro compound of the formula (24) is hydrogenated as described in Example 1. The amino compound produced (4.72 g) is dissolved in tetrahydrofuran (20 ml) and pyridine (10 ml), and 2,4-ditert.amylphenoxybutyryl chloride (3.12 g) is added. The solution obtained stands overnight at room temperature. Water (100 ml) is added, and the product extracted with ether, dried, and evaporated. 2.87 g of the compound of the formula (16) crystalline on triturating with petrol (80–100°C) (melting point 137–141°C).

Example 3:

A coating is prepared having the following coating weights:

| | |
|---|---|
| Compound of the formula (15) | 6 mg/dm² |
| dissolved in tricresylphosphate | 12 mg/dm² |
| with a conventional silver chloro-bromide photographic emulsion giving a silver coating weight of | 12 mg/dm² |
| in gelatin | 80 mg/dm². |

This is compared with a similar coating containing instead of compound (15) the conventional 4-equivalent magenta coupler of the formula

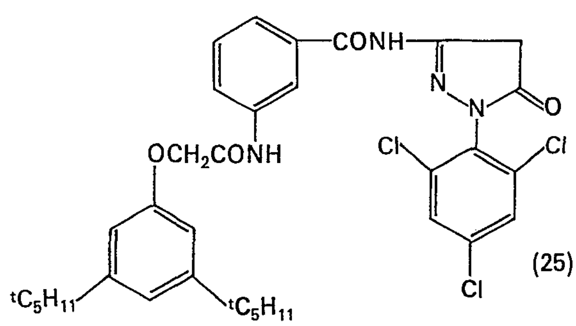

(25)

The coatings are fogged with light and then

developed at 38°C in a developing solution containing:

4-amino-3-methyl-N-ethyl-N-2-hydroxyethyl aniline sulfate (3.2 g), hydroxylamine sulfate (3 g), sodium carbonate (30 g), potassium bromide (1.8 g), potassium hydrogen sulfate (7 g), sodium bicarbonate (8 g), sodium sulfite (2 g) and sodium tripolyphosphate (2 g) and water to make up to 1 litre.

The coatings are bleached and fixed, and the densities measured:

Compound of the formula (15): density 1.57; max. 532 nm

Compound of the formula (25) (control): density 1.45; max. 555 nm.

These coatings are then exposed in a Xenotest machine for 50 hours. The reduction of density is as follows:

| | |
|---|---|
| Compound of the formula (15) | 9%, |
| Control compound | 16%. |

Different samples of the coatings are dipped in a 1% aqueous formaldehyde solution for 2 minutes and then processed as above. The reduction in density of the coating of compound (15) is about 3%, whereas with the control compound of the formula (25) practically no density remains.

Example 4:

3 g of the nitro compound of the formula (24) are hydrogenated as described in Example 1. The product is added to the acid chloride from γ-2,4-di-tert.amylphenoxy butyric acid (1.8 g) in tetrahydrofuran and the solution obtained stands at room temperature for 20 hours with pyridine. The resulting solution is extracted with ethylacetate and recrystallised from mixtures of petrol and ethylacetate, yielding 0.8 g of the product of the formula

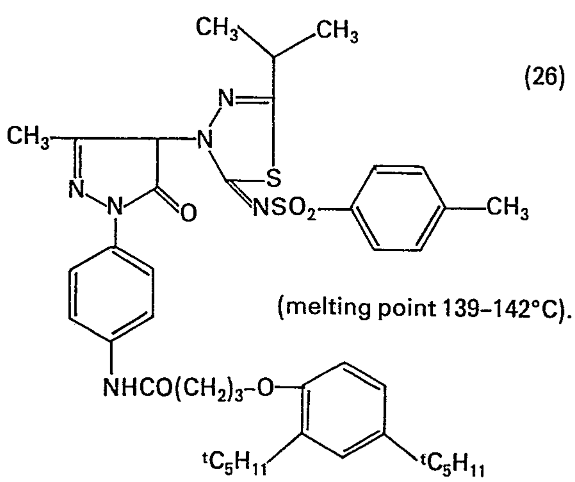

(26)

(melting point 139–142°C).

Example 5:

As described in Example 1. This compound has a melting point of 191–194°C.

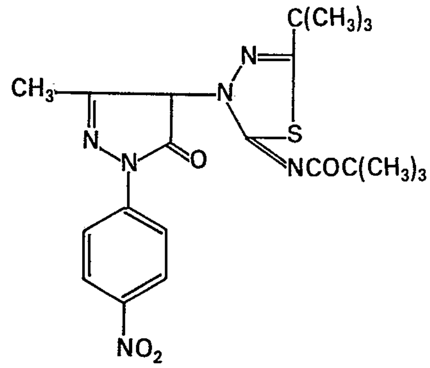

(27)

This compound (1.07 g) is hydrogenated in ethanol (50 ml) using palladium on charcoal as the catalyst. The solvent is then removed, and the residue dissolved in tetrahydrofuran (50 ml) and pyridine (0.5 ml) and di-tert.amylphenoxy acetic acid chloride (0.73 g) is added. The mixture stands at room temperature for 6 hours, and then diluted hydrochlorid acid (50 ml) is added. The product is crystallised from a methanol/ethanol (2:1) mixture giving 1.01 g of the compound of the formula

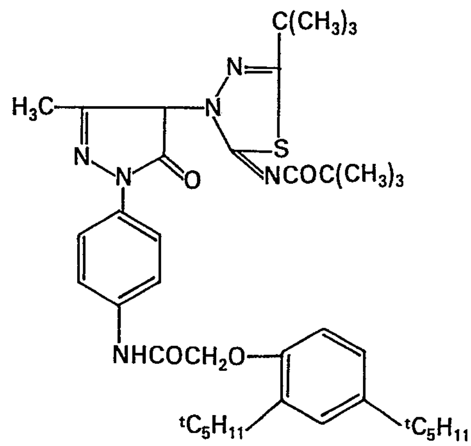

(28)

(melting point 115–119°C).

## Claims

1. A pyrazolone colour coupler of the formula

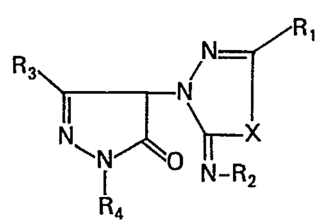

wherein X is -S-, -O- or -Se-, $R_1$ is hydrogen, chlorine, bromine or iodine or optionally substituted alkyl, phenyl, alkoxy, phenoxy, acyloxy, alkylthio or phenylthio, $R_2$ is -CO-alkyl, $-CO-C_6H_5$, $-CON-C_6H_5$, -CONH-alkyl, $-SO_2$-alkyl or $-SO_2-C_6H_5$, wherein the alkyl and $C_6H_5$-radicals are optionally substituted, $R_3$ is alkyl, phenyl, -NH-alkyl, $-NH-C_6H_5$, -NHCONH-alkyl,

-NHCONH-$C_6H_5$, -NHCO-$C_6H_5$ or -NHCO-alkyl, wherein the alkyl and $C_6H_5$-radicals are optionally substituted, $R_4$ is hydrogen or substituted alkyl or phenyl, at least in one of the alkyl or phenyl groups of $R_3$ or $R_4$ being present a ballasting group B.

2. A pyrazolone colour coupler according to claim 1, wherein $R_1$ is hydrogen, chlorine, bromine or iodine or alkyl, alkoxy or alkylthio, wherein the alkyl moiety contains 1 to 6 carbon atoms, or phenyl, phenoxy, phenylthio or carboxylic acid acyl, $R_2$ is -CO-alkyl, -CONH-alkyl or -$SO_2$-alkyl, wherein the alkyl moiety contains 1 to 6 carbon atoms, or -$C_6H_5$, -CO-$C_6H_5$, -CONH-$C_6H_5$ or -$SO_2$-$C_6H_5$, and wherein the alkyl and phenyl moieties are unsubstituted or substituted by alkyl or hydroxyalkyl, carbalkoxy, -NHCO-alkyl or halogen, the alkyl radicals containing 1 to 4 carbon atoms, $R_3$ is alkyl, -NH-alkyl or -NHCONH-alkyl, the alkyl moieties containing 1 to 6 carbon atoms, or phenyl, -NH-$C_6H_5$, -NHCONH-$C_6H_5$ or -NHCO-$C_6H_5$, wherein phenyl and the alkyl moieties are unsubstituted or substituted by halogen, cyano, hydroxyalkyl, carbalkoxy, -CONH-alkyl or -NHCO-alkyl, the alkyl radicals containing 1 to 6 carbon atoms, $R_4$ is hydrogen, alkyl having 1 to 6 carbon atoms or phenyl, where the alkyl moieties and phenyl are substituted by halogen, alkyl, hydroxyalkyl or carbalkoxy, the alkyl moiety containing 1 to 4 carbon atoms, or -$SO_2NHR_5$, -$NHCOR_5$ or -$NHSO_2R_5$, wherein $R_5$ is alkyl having 1 to 6 carbon atoms or phenyl, which radicals are unsubstituted or substituted by methoxy, ethoxy or halogen, at least in one of the alkyl or phenyl groups of $R_3$ or $R_4$ being present a ballasting group B, and X is as defined in claim 1.

3. A pyrazolone colour coupler according to claim 2, wherein X is -O- or -S-, $R_1$ is alkyl or alkoxy, wherein the alkyl moiety contains 1 to 6 carbon atoms, or phenyl or phenoxy, $R_2$ is -CO-alkyl or -$SO_2$-phenyl, wherein phenyl and the alkyl moieties are unsubstituted or substituted by alkyl or hydroxyalkyl each having 1 to 4 carbon atoms, or halogen, $R_3$ is alkyl, phenyl, -NH-$C_6H_5$, -NHCONH-$C_6H_5$ or -NHCO-$C_6H_5$, wherein phenyl and the alkyl moieties are unsubstituted or substituted by halogen, cyano, hydroxyalkyl, carbalkoxy, -CONH-alkyl or -NHCO-alkyl, the alkyl radicals containing 1 to 6 carbon atoms, $R_4$ is phenyl substituted by halogen, -$SO_2NHR_5$, -$NHCOR_5$ or -$NHSO_2R_5$, wherein $R_5$ is alkyl having 1 to 6 carbon atoms, at least in one of the alkyl or phenyl groups of $R_3$ or $R_4$ being present a ballasting group B.

4. A pyrazolone colour coupler according to claim 3, wherein B is alkyl having more than 10 carbon atoms or aryloxy substituted by tertiary alkyl groups having 4 to 8 carbon atoms.

5. A pyrazolone colour coupler according to claim 4, wherein B is alkyl having 10 to 30 carbon atoms or phenoxy substituted by tertiary pentyl groups.

6. A pyrazolone colour coupler according to claim 3 wherein X is -S-.

7. A process for the preparation of a pyrazolone colour coupler according to claim 1 which comprises reacting a pyrazolone of the formula

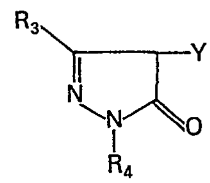

wherein $R_3$ and $R_4$ are as defined in claim 1 and Y is chlorine or bromine, with an oxa- thia- or selenadiazole of the formula

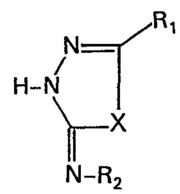

wherein X, $R_1$ and $R_2$ are as defined in claim 1, in the presence of a base either in a melt or in solution or suspension in a solvent.

8. A process for the preparation of a pyrazolone colour coupler according to claim 1 which comprises reacting a compound of the formula

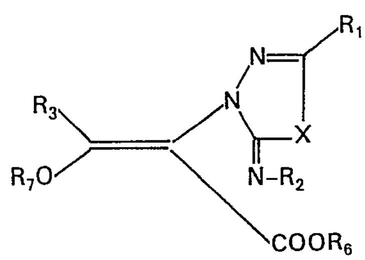

wherein $R_6$ and $R_7$ are each hydrogen, alkyl or phenyl, and $R_1$, $R_2$, $R_3$ and X are as defined in claim 1 or groups which may be transformed into such groups, with a hydrazine of the formula

$H_2N-NHR_4$

wherein $R_4$, has the meaning assigned to it in claim 1, or is a group which may be transformed into such a group either in a melt or in a solvent, optionally in the presence of an acidic catalyst or a basic catalyst, at ambient or higher temperature.

9. A process according to claim 8, wherein the reaction is carried out in the presence of an acidic catalyst.

10. A process according to claim 9, wherein the acidic catalyst is acetic acid.

11. A process according to claim 8, wherein the process is carried out in a solvent.

12. A process according to claim 11, wherein the solvent is an unsubstituted or substituted alcohol.

13. A process according to claim 12, wherein the process is carried out at the boiling point temperature of the alcohol used.

14. Colour photographic silver halide material which comprises in at least one silver halide layer a pyrazolone colour coupler according to claim 1.

**Patentansprüche**

1. Pyrazolonfarbkuppler der Formel

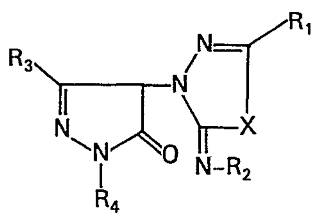

worin X -S-, -O- oder -Se-, $R_1$ Wasserstoff, Chlor, Brom oder Jod oder gegebenenfalls substituiertes Alkyl, Phenyl, Alkoxy, Phenoxy, Acyloxy, Alkylthio oder Phenylthio, $R_2$ -CO-Alkyl, -CO-$C_6H_5$, -CONH-$C_6H_5$, -CONH-Alkyl, -$SO_2$-Alkyl oder -$SO_2$-$C_6H_5$, worin die Alkyl- und $C_6H_5$-Reste gegebenenfalls substituiert sind, $R_3$ Alkyl, Phenyl, -NH-Alkyl, -NH-$C_6H_5$, -NHCONH-Alkyl, -NHCONH-$C_6H_5$, -NHCO-$C_6H_5$ oder -NHCO-Alkyl, worin die Alkyl- und $C_6H_5$-Reste gegebenenfalls substituiert sind, $R_4$ Wasserstoff oder substituiertes Alkyl oder Phenyl ist, wobei in mindestens einer der Alkyl- oder Phenylgruppen $R_3$ oder $R_4$ eine Ballastgrupe B enthalten ist.

2. Pyrazolonfarbkuppler nach Anspruch 1, worin $R_1$ Wasserstoff, Chlor, Brom oder Jod oder Alkyl, Alkoxy oder Alkylthio, worin der Alkylrest 1 bis 6 Kohlenstoffatome enthält, oder Phenyl, Phenoxy, Phenylthio oder Carbonsäureacyl, $R_2$ -CO-Alkyl, -CONH-Alkyl oder -$SO_2$-Alkyl, worin der Alkylrest 1 bis 6 Kohlenstoffatome enthält, oder -$C_6H_5$, -CO-$C_6H_5$, -CONH-$C_6H_5$ oder -$SO_2$-$C_6H_5$, worin die Alkyl- und Phenylreste unsubstituiert oder substituiert sind durch Alkyl oder Hydroxyalkyl, Carbalkoxy, -NHCO-Alkyl oder Halogen, wobei die Alkylreste 1 bis 4 Kohlenstoffatome enthalten, $R_3$ Alkyl, -NH-Alkyl oder -NHCONH-Alkyl, worin die Alkylreste 1 bis 6 Kohlenstoffatome enthalten, oder Phenyl, -NH-$C_6H_5$ -NHCONH-$C_6H_5$ oder -NHCO-$C_6H_5$, worin die Phenyl- und Alkylreste unsubstituiert oder mit Halogen, Cyano, Hydroxyalkyl, Carbalkoxy, -CONH-Alkyl oder -NHCO-Alkyl substituiert sind, wobei die Alkylreste 1 bis 6 Kohlenstoffatome enthalten, $R_4$ Wasserstoff, Alkyl mit 1 bis 6 Kohlenstoffatomen oder Phenyl, worin die Alkylreste und Phenyl mit Halogen, Alkyl, Hydroxyalkyl oder Carbalkoxy substituiert sind, wobei die Alkylreste 1 bis 4 Kohlenstoffatome enthalten, oder -$SO_2NHR_5$, -$NHCOR_5$ oder -$NHSO_2R_5$ ist, worin Alkyl mit 1 bis 6 Kohlenstoffatomen oder Phenyl ist, wobei diese Reste unsubstituiert oder substituiert sind mit Methoxy, Äthoxy oder Halogen, wobei in mindestens einer der Alkyl- oder Phenylgruppen $R_3$ oder $R_4$ eine Ballastgruppe B enthalten ist und X die in Anspruch 1 angegebene Bedeutung hat.

3. Pyrazolonfarbkuppler nach Anspruch 2, worin X -O- oder -S-, $R_1$ Alkyl oder Alkoxy, worin der Alkylrest 1 bis 6 Kohlenstoffatome enthält, oder Phenyl oder Phenoxy, $R_2$ -CO-Alkyl oder -$SO_2$-Phenyl, worin Phenyl und die Alkylreste unsubstituiert oder substituiert sind mit Alkyl oder Hydroxyalkyl mit je 1 bis 4 Kohlenstoffatomen, oder Halogen, $R_3$ Alkyl, Phenyl, -NH-$C_6H_5$ -NHCONH-$C_6H_5$ oder -NHCO-$C_6H_5$, worin Phenyl und die Alkylreste unsubstituiert oder substituiert sind mit Halogen, Cyano, Hydroxyalkyl, Carbalkoxy, -CONH-Alkyl oder -NHCO-Alkyl, wobei die Alkylreste 1 bis 6 Kohlenstoffatome enthalten, $R_4$ Phenyl substituiert mit Halogen, -$SO_2NHR_5$ oder -$NHSO_2R_5$ ist, worin $R_5$ Alkyl mit 1 bis 6 Kohlenstoffatomen ist, wobei in mindestens einer der Alkyl- oder Phenylgruppen $R_3$ oder $R_4$ eine Ballastgruppe B enthalten ist.

4. Pyrazolonfarbkuppler nach Anspruch 3, worin B Alkyl mit mehr als 10 Kohlenstoffatomen oder Aryloxy, substituiert mit tertiären Alkylgruppen mit 4 bis 8 Kohlenstoffatomen, ist.

5. Pyrazolonfarbkuppler nach Anspruch 4, worin B Alkyl mit 10 bis 30 Kohlenstoffatomen oder Phenoxy, substituiert mit tertiären Pentylgruppen, ist.

6. Pyrazolonfarbkuppler nach Anspruch 3, worin X -S- ist.

7. Verfahren zur Herstellung von Pyrazolonfarbkupplern nach Anspruch 1, dadurch gekennzeichnet, dass man ein Pyrazolon der Formel

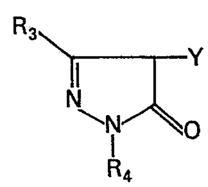

worin $R_3$ und $R_4$ die in Anspruch 1 angegebenen Bedeutungen haben und Y Chlor oder Brom ist, mit einem Oxa-, Thia- oder Selenadiazol der Formel

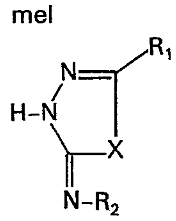

worin X, $R_1$ und $R_2$ die in Anspruch 1 angegebene Bedeutung haben, in Gegenwart einer Base entweder in einer Schmelze oder in Lösung oder in einer Suspension in einem Lösungsmittel umsetzt.

8. Verfahren zur Herstellung von Pyrazolonfarbkupplern nach Anspruch 1, dadurch gekennzeichnet, dass man eine Verbindung der Formel

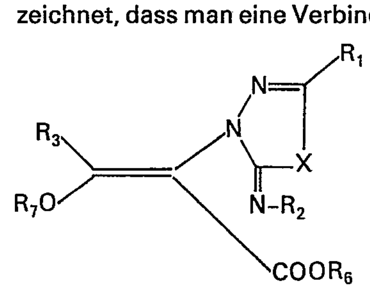

worin $R_6$ und $R_7$ Wasserstoff, Alkyl oder Phenyl sind und $R_1$, $R_2$, $R_3$ und X die in Anspruch 1 angegebene Bedeutung haben oder Gruppen sind, die in solcher Substituenten überführt werden können, mit einem Hydrazin der Formel

$H_2N\text{-}NHR_4$

worin $R_4$ die in Anspruch 1 angegebene Bedeutung hat oder eine Gruppe ist, die in solche Substituenten überführt werden kann, in einer Schmelze, oder in einem Lösungsmittel, gegebenenfalls in Anwesenheit eines sauren oder basischen Katalysators bei Raumtemperatur oder erhöhter Temperatur umsetzt.

9. Verfahren nach Anspruch 8, worin die Reaktion in Gegenwart eines sauren Katalysators ausgeführt wird.

10. Verfahren nach Anspruch 9, worin der saure Katalysator Essigsäure ist.

11. Verfahren nach Anspruch 8, worin die Umsetzung in einem Lösungsmittel ausgeführt wird.

12. Verfahren nach Anspruch 11, worin das Lösungsmittel ein unsubstituierter oder substituierter Alkohol ist.

13. Verfahren nach Anspruch 12, worin die Umsetzung bei der Siedetemperatur des verwendeten Alkohols ausgeführt wird.

14. Farbphotographisches Silberhalogenidmaterial, das in mindestens einer Silberhalogenidschicht einen Pyrazolonfarbkuppler nach Anspruch 1 enthält.

**Revendications**

1. Coupleur de couleurs pyrazolone de formule:

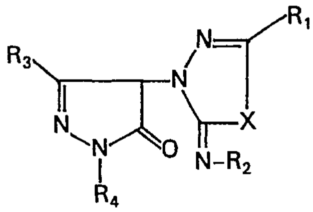

dans laquelle X est -S-, -O- ou -Se-, $R_1$ est un atome d'hydrogène, de chlore, de brome ou de iode, ou un groupe, éventuellement substitué, alkyle, phényle, alcoxy, phénoxy, acyloxy, alkylthio ou phénylthio, $R_2$ est un groupe -CO-alkyle, $-CO-C_6H_5$, $-CONH-C_6H_5$, -CONH-alkyle, $-SO_2$-alkyle ou $-SO_2-C_6H_5$, où les radicaux alkyle et $C_6H_5$ sont éventuellement substitués, $R_3$ est un groupe alkyle, phényle, -NH-alkyle, $-NH-C_6H_5$, -NHCONH-alkyle, $-NHCONH-C_6H_5$, $-NHCO-C_6H_5$ ou -NHCO-alkyle, où les radicaux alkyle et $C_6H_5$ sont éventuellement substitués, $R_4$ est un atome d'hydrogène ou un groupe alkyle ou phényle substitué, un groupe alourdissant B étant présent au moins dans l'un des groupes alkyle ou phényle de $R_3$ ou $R_4$.

2. Coupleur de couleurs pyrazolone conforme à la revendication 1, dans lequel $R_1$ est un atome d'hydrogène, de chlore, de brome ou d'iode, ou un groupe alkyle, alcoxy ou alkylthio, où le fragment alkyle contient de 1 à 6 atomes de carbone, ou un groupe phényle, phénoxy, phénylthio ou un groupe acyle d'acide carboxylique, $R_2$ est un groupe -CO-alkyle, -CONH-alkyle, ou $-SO_2$-alkyle, où le fragment alkyle contient de 1 à 6 atoms de carbone, ou $-C_6H_5$, $-CO-C_6H_5$, $-CONH-C_6H_5$ ou $-SO_2-C_6H_5$, et où les fragments alkyle et phényle ne sont pas substitués ou bien sont substitués par des groupes alkyle, hydroxyalkyle, carbalcoxy, -NHCO-alkyle ou par des atomes d'halogène, les radicaux alkyle contenant de 1 à 4 atomes de carbone, $R_3$ est un groupe alkyle, -NH-alkyle ou -NHCONH-alkyle, les fragments alkyle contenant de 1 à 6 atomes de carbone, ou un groupe phényle, $-NH-C_6H_5$, $-NHCONH-C_6H_5$ ou $-NHCO-C_6H_5$, où les fragments phényle et alkyle ne sont pas substitués ou bien sont substitués par des atomes d'halogène ou par des groupes cyano, hydroxyalkyle, carbalcoxy, -CONH-alkyle ou -NHCO-alkyle, les radicaux alkyle contenant de 1 à 6 atomes de carbone, $R_4$ est un atome d'hydrogène ou un groupe alkyle ayant de 1 à 6 atomes de carbone ou un groupe phényle, où les fragments alkyle et phényle sont substitués par des atomes d'halogène ou par des groupes alkyle, hydroxyalkyle, ou carbalcoxy, le fragment alkyle contenant de 1 à 4 atomes de carbone, ou par des groupes $-SO_2NHR_5$, $-NHCOR_5$ ou $-NHSO_2R_5$, où $R_5$ est un groupe alkyle ayant de 1 à 6 atomes de carbone ou un groupe phényle, lesquels groupes ne sont pas substitués ou bien sont substitués par un groupe méthoxy, éthoxy ou par des atomes d'halogène, un groupe alourdissant B étant présent au moins dans l'un des groupes alkyle ou phényle de $R_3$ ou $R_4$, et X est tel que défini dans la revendication 1.

3. Coupleur de couleurs pyrazolone conforme à la revendication 2, dans lequel X est -O- ou -S-, $R_1$ est un groupe alkyle ou alcoxy où le fragment alkyle contient de 1 à 6 atomes de carbone, ou un groupe phényle ou phénoxy, $R_2$ est un groupe -CO-alkyle ou $-SO_2$-phényle, où les fragments phényle et alkyle ne sont pas substitués ou bien sont substitués par des groupes alkyle ou hydroxyalkyle ayant chacun de 1 à 4 atomes de carbone, ou par des atomes d'halogène, $R_3$ est un groupe alkyle, phényle, $-NH-C_6H_5$, $-NHCONH-C_6H_5$ ou $-NHCO-C_6H_5$, où les fragments alkyle et phényle ne sont pas substitués ou bien sont substitués par des atomes d'halogène ou par des groupes cyano, hydroxyalkyle, carbalcoxy, -CONH-alkyle ou -NHCO-alkyle, les radicaux alkyle contenant de 1 à 6 atomes de carbone, $R_4$ est un groupe phényle substitue par des atomes d'halogène ou par des groupes $-SO_2NHR_5$, $-NHCOR_5$ ou $-NHSO_2R_5$, où $R_5$ est un groupe alkyle ayant de 1 à 6 atomes de carbone, un groupe alourdissant B étant présent au moins dans l'un des groupes alkyle ou phényle de $R_3$ ou $R_4$.

4. Coupleur de couleurs pyrazolone conforme à la revendiction 3, où B est un groupe alkyle ayant plus de 10 atomes de carbone, ou un groupe aryloxy substitué par des groupes alkyle tertiaires ayant de 4 à 8 atomes de carbone.

5. Coupleur de couleurs pyrazolone conforme à la revendiction 4, où B est un groupe alkyle ayant de 10 à 30 atomes de carbone, ou un groupe phénoxy substitué par des groupes pentyle tertiaires.

6. Coupleur de couleurs pyrazolone conforme à la revendication 3, où X est -S-.

7. Procédé de préparation d'un coupleur de couleurs pyrazolone conforme à la revendication 1, qui consiste à faire réagir une pyrazolone de formule:

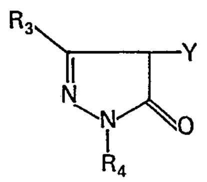

où $R_3$ et $R_4$ sont tels que définis dans la revendiction 1, et Y est un atome de chlore ou de brome, avec un oxadiazole, un thiadinazole, ou un sélénadiazole, de formule:

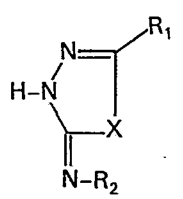

où X, $R_1$ et $R_2$ sont tels que définis dans la revendication 1, en présence d'une base, soit à l'état fondu, soit en solution ou en suspension dans un solvant.

8. Procédé de préparation d'un coupleur de couleurs pyrazolone conforme à la revendication 1, qui consiste à faire réagir un composé de formule:

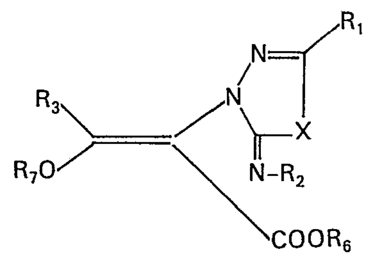

où $R_6$ et $R_7$ sont chacun un atome d'hydrogène ou un groupe alkyle ou phényle, et $R_1$, $R_2$, $R_3$ et X sont tels que définis dans la revendication 1 ou des groupes qui peuvent être transformés en de tels groupes, avec une hydrazine de formule:

$H_2N\text{-}NHR_4$

où $R_4$ a la signification qui lui a été assignée dans la revendication 1, ou bien est un groupe qui peut être transformé en un tel groupe, soit à l'état fondu, soit dans un solvant, éventuellement en présence d'un catalyseur acide ou d'un catalyseur basique, à température ambiante ou à température supérieure.

9. Procédé conforme à la revendication 8, dans lequel la réaction est effectuée en présence d'un catalyseur acide.

10. Procédé conforme à la revendication 9, dans lequel le catalyseur acide est l'acide acétique.

11. Procédé conforme à la revendication 8, dans lequel le procédé est effectué dans un solvant.

12. Procédé conforme à la revendication 11, dans lequel le solvant est un alcool substituée ou non substitué.

13. Procédé conforme à la revendication 12, dans lequel le procédé est réalisé à la température du point d'ébullition de l'alcool utilisé.

14. Matériau d'halogénure d'argent pour photographies en couleurs qui comporte dans au moins une couche d'halogénure d'argent un coupleur de couleurs pyrazolone conforme à la revendication 1.